# EUROPEAN PATENT APPLICATION

(11) **EP 4 702 973 A1**
(43) Date of publication of application: **04.03.2026**
(21) Application number: 23848463.8
(22) Date of filing: 25.05.2023
(51) Int. Cl.: A61K 9/08, A61K 31/438, A61K 47/40, A61P 27/02, A61P 29/00

(54) **OPHTHALMIC COMPOSITION FOR TREATING NON-INFECTIOUS INFLAMMATORY DISEASES**

(30) Priority: 28.04.2023 CN 202310484101
(71) Applicant: Vivavision Biotech Ltd., Pudong New Area, Shanghai 201203 (CN)
(72) Inventor: XIA, Erning, Shanghai 201203 (CN); HAN, Qiao, Shanghai 201203 (CN); SHEN, Wang, Shanghai 201203 (CN); LI, Yong, Shanghai 201203 (CN); GAO, Hongyan, Shanghai 201203 (CN)
(74) Representative: Dantz, Dirk
(86) International application number: PCT/CN2023/096225
(87) International publication number: WO 2024/221524

(57) **Abstract**

The present application provides an ophthalmic composition for the treatment of a non-infectious inflammatory disease, wherein the ophthalmic composition comprises an active ingredient and an ophthalmic excipient, wherein the active ingredient is a JAK inhibitor; and the ophthalmic excipient comprises a pH buffering agent, an tonicity agent or osmolyte, a solubilizer and water for injection. The ophthalmic composition using the composition and content of the present application for topical eye drop administration is compatible with the eye and stable, tear friendly and comfortable, can improve the bioavailability of the active ingredient in the ophthalmic composition, can be used for the treatment of a non-infectious inflammatory disease and have good therapeutic effects. Additionally, the ophthalmic composition is less invasive and has fewer side effects, while its production process is simple which facilitates mass production.

## Description

The present application claims benefit of priority of Chinese Patent Application No. 202310484101.X, filed before the CNIPA on April 28, 2023, titled "OPHTHALMIC COMPOSITION FOR TREATING NON-INFECTIOUS INFLAMMATORY DISEASES", which is incorporated herein by reference in its entirety.

### TECHNICAL FIELD

The present application relates to the field of pharmaceutical technology, in particular to an ophthalmic composition comprising a JAK inhibitor for the treatment of a non-infectious inflammatory disease.

### BACKGROUND

The eye is one of the most important organs in the human body, and any dysfunction in vision can have a serious impact on daily activities and life. Uveitis, a vision-threatening inflammation of the uvea, is very common worldwide, with anterior uveitis being one of the most common intraocular inflammations. Recurrent or untreated anterior uveitis has potentially serious consequences. Anterior uveitis, an inflammation of the middle layer (including the iris and adjacent tissues) of the eye, is either infectious or non-infectious, and may be associated with autoimmune or inflammatory diseases.

Janus kinase (JAK) is a family of intracellular, non-receptor tyrosine kinases with four members, namely JAK1, JAK2, JAK3, and tyrosine-protein kinase 2 (TYK2), which mediates cytokine-producing signaling through JAK-signal transducer and activation of transcription (STAT) signaling pathway, and is involved in many important biological processes such as cell proliferation, differentiation, apoptosis, and immune regulation. This pathway is a common signaling pathway in vertebrates, through which many important cytokines including IL, IFN, granulocyte/macrophage colony-stimulating factor, erythropoietin and thrombopoietin transmit signals. Thus, the JAK-STAT pathway is closely related to the hematologic and immune systems and plays an important role in immune-mediated inflammatory diseases such as atopic dermatitis, psoriasis, psoriatic arthritis, rheumatoid arthritis, alopecia areata, leukoplakia, and non-infectious uveitis. Therefore, the purpose of the treatment of non-infectious inflammatory diseases such as non-infectious uveitis can be achieved by blocking the JAK-STAT pathway and reducing the expression of pro-inflammatory cytokines through inhibiting the activity of JAK.

Currently, chronic uveitis is mainly treated with topical corticosteroid eye drops. Corticosteroids as standard and first-line treatments for uveitis can be administered ophthalmically via eye drops or injections and are designed to reduce, control, or mitigate inflammation. A number of corticosteroid eye drops are available, such as 0.125% and 1% prednisolone acetate, 1% betamethasone, 0.1% dexamethasone sodium phosphate (also available in a 0.05% ointment form), 0.1% and 0.25% fluoromethalone (also available in a 0.1% ointment form), 0.5% loteprednol (also available in a 0.5% ointment form) and 1% rimexolone. However, prolonged use of corticosteroid eye drops can greatly increase intraocular pressure and also increase the risk of cataract. Therefore, there is an urgent need to develop a medicament that is less invasive, has fewer side effects or sequelae, is an alternative to corticosteroids, and is more effective in non-infectious inflammatory diseases such as uveitis.

### SUMMARY

The present application aims to provide an ophthalmic composition that is compatible with the eye and stable, administered topically by eye drops, for use in the treatment of non-infectious inflammatory diseases with good effect and less side effects. Specific embodiments are provided as follows.

A first aspect of the present application provides an ophthalmic composition comprising an active ingredient and an ophthalmic excipient, wherein the active ingredient is a JAK inhibitor, and the JAK inhibitor is at least one selected from the group consisting of VVN461, tofacitinib, ruxolitinib, baricitinib, peficitinib, delgocitinib, upadacitinib, filgotinib, abrocitinib, deucravacitinib, ritlecitinib, brepocitinib, jaktinib, ivarmacitinib (SHR0302), itacitinib (IBI-377), golidocitinib (AZD4205), KL130008, TLL018 and LNK01001; preferably VVN461; the VVN461 is represented by formula I;
the active ingredient accounts for 0.01 wt% to 5 wt%, preferably 0.1 wt% to 2 wt%, more preferably 0.1 wt% to 1 wt% of the ophthalmic composition; 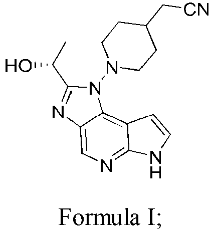
the ophthalmic excipient comprises a pH buffering agent, an tonicity agent or osmolyte, a solubilizer and water for injection; the pH buffering agent accounts for 0.001 wt% to 2.5 wt%, preferably 0.01 wt% to 1.5 wt%, more preferably 0.1 wt% to 0.25 wt% of the ophthalmic composition;
the tonicity agent or osmolyte accounts for 0.01 wt% to 2.5 wt%, preferably 0.2 wt% to 2 wt% of the ophthalmic composition;
the solubilizer accounts for 0.5 wt% to 15 wt%, preferably 1 wt% to 12 wt% of the ophthalmic composition; and
the ophthalmic composition has a pH of 4 to 8, preferably 5 to 7; and an osmolality of 200 mOsmo/Kg to 400 mOsmo/Kg, preferably 240 mOsmo/Kg to 380 mOsmo/Kg, more preferably 240 mOsmo/Kg to 320 mOsmol/Kg.
In some embodiments of the present application, the pH buffering agent is any one selected from the group consisting of boric acid-borate, citric acid-citrate, acetic acid-sodium acetate, trometamol-hydrochloric acid, sodium bicarbonate and phosphate;
the borate is at least one selected from the group consisting of sodium borate, potassium borate and hydrate thereof;
the citrate is at least one selected from the group consisting of potassium citrate, sodium citrate, disodium hydrogen citrate, sodium dihydrogen citrate, dipotassium hydrogen citrate, potassium dihydrogen citrate and hydrate thereof; and
the phosphate is at least one selected from the group consisting of disodium hydrogen phosphate, sodium dihydrogen phosphate, dipotassium hydrogen phosphate, potassium dihydrogen phosphate and hydrate thereof.
In some embodiments of the present application, the tonicity agent or osmolyte is an inorganic tonicity agent or osmolyte and/or an organic tonicity agent or osmolyte;
the inorganic tonicity agent or osmolyte is at least one selected from the group consisting of sodium chloride, potassium chloride, calcium chloride, zinc chloride and magnesium chloride; and
the organic tonicity agent or osmolyte is at least one selected from the group consisting of glucose, glycerol, propylene glycol, glycine, diglycine, alanine, taurine, tetrahydromethylpyrimidine carboxylic acid, erythritol, mannitol, sorbitol and trehalose.

In some embodiments of the present application, the solubilizer is a cyclodextrin, and the cyclodextrin is at least one selected from the group consisting of alpha-cyclodextrin, beta-cyclodextrin, gamma-cyclodextrin, sulfobutyl-beta-cyclodextrin, hydroxypropyl-beta-cyclodextrin and hydroxypropyl-gamma-cyclodextrin.

In some embodiments of the present application, the ophthalmic excipient further comprises a mucoadhesive agent; the mucoadhesive agent is at least one selected from the group consisting of polyvinylpyrrolidone (PVP), polyvinyl alcohol, methylcellulose, hydroxypropylmethylcellulose (HPMC), carboxymethylcellulose (CMC), sodium hyaluronate, sodium alginate, polyethylene glycol (PEG), thiolated polyacrylic acid (PAA-SH), poloxamer, poloxamine, Mrij (polyoxyethylene ether oleate), Brij (fatty alcohol polyoxyethylene ether), cellulose acetate phthalate (CAP), hydroxyethyl cellulose (HEC), poly(amidoamine) dendrimer (PAMAM), poly(dimethylsiloxane) (PDMS) and hydroxypropyl guar (HP-Guar); and
the mucoadhesive agent accounts for 0.001 wt% to 15 wt%, preferably 0.005 wt% to 10 wt%, more preferably 0.01 wt% to 5 wt% of the ophthalmic composition.

In some embodiments of the present application, the ophthalmic excipient further comprises a surfactant; the surfactant is at least one selected from the group consisting of sodium dodecyl sulfate, polyethoxylated sorbitan fatty acid ester, polyoxyethylene alkyl ether, polyoxyethylene stearate, poloxamine, sorbitan fatty acid ester, polyethylene glycol, polyethoxylated fatty alcohol, PEG-40 hydrogenated castor oil, docusate sodium, quaternary ammonium compound, C₆-C₂₀ fatty acid, saccharide-fatty acid ester, fatty acid glyceride, polysorbate, poloxamer and tyloxapol; and
the surfactant accounts for 0.01 wt% to 5 wt% of the ophthalmic composition.

In some embodiments of the present application, the ophthalmic excipient further comprises a comforting agent; the comforting agent is at least one selected from the group consisting of polyol, a cellulose derivative, dextran, polyethylene glycol, polysorbate, povidone, trehalose, hyaluronic acid, sodium hyaluronate and sodium alginate;
the polyol is at least one selected from the group consisting of glycerol, propylene glycol, polyvinyl alcohol and mannitol;
the cellulose derivative is at least one selected from the group consisting of hydroxypropylmethylcellulose-E4M, hydroxypropylmethylcellulose-LV, hydroxyethylcellulose, hydroxymethylcellulose, methylcellulose, hemicellulose and ethylcellulose; and
the comforting agent accounts for 0.001 wt% to 15 wt%, preferably 0.01 wt% to 5 wt% of the ophthalmic composition.

In some embodiments of the present application, the ophthalmic excipient further comprises a preservative; the preservative is at least one selected from the group consisting of benzalkonium chloride, sorbic acid, disodium edetate, boric acid, sodium borate, sodium bisulfate, sodium thiosulfate, ascorbate, urea peroxide, benzalkonium bromide, sodium chlorite and polyquaternium-1; and
the preservative accounts for 0.01 wt% to 0.05 wt% of the ophthalmic composition.

In some embodiments of the present application, the ophthalmic excipient further comprises an antioxidant; the antioxidant is at least one selected from the group consisting of sodium thiosulfate, sodium metabisulfite, N-acetylcysteine, butylhydroxyanisole (BHA) and butylhydroxytoluene (BHT); and
the antioxidant accounts for 0.01 wt% to 5 wt%, preferably 0.1 wt% to 0.5 wt% of the ophthalmic composition.

In some embodiments of the present application, the ophthalmic excipient further comprises a chelating agent; the chelating agent is at least one selected from the group consisting of nitrilotriacetic acid, ethylenediamine disuccinic acid, iminodisuccinic acid, methylglycine diacetic acid, L-glutamic acid N,N-diacetic acid, ethylenediamine N,N'-diglutamic acid, ethylenediamine N,N'-bis(malonic acid), 3-hydroxy-2,2-iminodisuccinic acid, 2-hydroxyethyliminodiacetic acid, pyridine-2,6-dicarboxylic acid, diethylenetriaminepentaacetic acid, hydroxyethyldiaminetriacetic acid, 1,2-diaminocyclohexanetetraacetic acid, hydroxyethylaminodiacetic acid, polyphosphate, citric acid and citrate, tartaric acid and tartrate, ethylenediaminetetraacetic acid and ethylenediaminetetraacetic acid disodium and hexametaphosphoric acid alkali metal salt; and
the chelating agent accounts for 0.001 wt% to 1 wt%, preferably 0.1 wt% to 0.25 wt% of the ophthalmic composition.

A second aspect of the present application provides use of the ophthalmic composition provided according to the first aspect of the present application in the preparation of a medicament for the treatment of a non-infectious inflammatory disease.

In some embodiments of the present application, the non-infectious inflammatory disease comprises uveitis; and the uveitis is at least one selected from the group consisting of anterior uveitis, choroiditis, iridocyclitis, intermediate uveitis, iritis, panuveitis, pars planitis, posterior uveitis and retinitis.

The present application provides an ophthalmic composition comprising a JAK inhibitor for the treatment of a non-infectious inflammatory disease. The ophthalmic composition using the composition and content of the present application for topical eye drop administration is compatible with the eye and stable, tear friendly and comfortable, can improve the bioavailability of the active ingredient in the ophthalmic composition, can be used for the treatment of a non-infectious inflammatory disease and have good therapeutic effects. Additionally, the ophthalmic composition is less invasive and has fewer side effects, while its production process is simple and facilitates mass production.

### DESCRIPTION OF THE DRAWINGS

In order to illustrate the examples of the present application or the technical solution of the prior art more clearly, a brief description of the drawings that need to be used in the examples or the prior art is provided as follows. It is obvious that the drawings in the following description are only some of the examples of the present invention and according to these drawings, other examples can be obtained by those skilled in the art without any inventive efforts.
FIG. 1 shows the results of anterior chamber inflammatory cell score in an experimental anterior uveitis model after eye drop administration of the ophthalmic compositions of the present application;
FIG. 2 shows the results of conjunctival congestion score in an experimental anterior uveitis model after eye drop administration of the ophthalmic compositions of the present application;
FIG. 3 shows the mechanism of action of VVN461 for the treatment of a non-infectious inflammatory disease.

### DETAILED DESCRIPTION

The technical solutions in the examples of the present application will be clearly and completely described below in conjunction with the accompanying drawings in the examples of the present application. It is obvious that the described examples are only a part of the embodiments of the present application and not all of them. All other embodiments obtained by a person of ordinary skill in the art based on the present application are within the scope of protection of the present application.

A first aspect of the present application provides an ophthalmic composition comprising an active ingredient and an ophthalmic excipient, wherein the active ingredient is a JAK inhibitor, and the JAK inhibitor is at least one selected from, but not limited to, the group consisting of compounds VVN461, tofacitinib, ruxolitinib, baricitinib, peficitinib, delgocitinib, upadacitinib, filgotinib, abrocitinib, deucravacitinib, ritlecitinib, brepocitinib, jaktinib, ivarmacitinib, itacitinib, golidocitinib, KL130008, TLL018 and LNK01001; preferably the JAK1/TYK2 dual inhibitor VVN461; the VVN461 is represented by formula I;
the active ingredient accounts for 0.01 wt% to 5 wt%, preferably 0.1 wt% to 2 wt%, more preferably 0.1 wt% to 1 wt% of the ophthalmic composition; 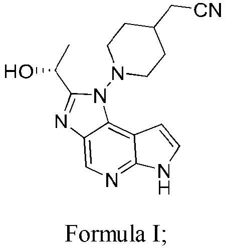
the ophthalmic excipient comprises a pH buffering agent, an tonicity agent or osmolyte, a solubilizer and water for injection; the pH buffering agent accounts for 0.001 wt% to 2.5 wt%, preferably 0.01 wt% to 1.5 wt%, more preferably 0.1 wt% to 0.25 wt% of the ophthalmic composition;
the tonicity agent or osmolyte accounts for 0.01 wt% to 2.5 wt%, preferably 0.2 wt% to 2 wt% of the ophthalmic composition;
the solubilizer accounts for 0.5 wt% to 15 wt%, preferably 1 wt% to 12 wt% of the ophthalmic composition; and
wherein the ophthalmic composition has a pH of 4 to 8, preferably 5 to 7; and an osmolality of 200 mOsmo/Kg to 400 mOsmo/Kg, preferably 240 mOsmo/Kg to 380 mOsmo/Kg, more preferably 240 mOsmo/Kg to 320 mOsmol/Kg.

In the present application, the compound VVN461 represented by formula I, has a molecular weight of 324.38, chemical name: (R)-2-(1-(2-(1-hydroxyethyl)imidazo[4,5-d]pyrrolo[2,3-b]pyridin-1(6H)-yl)piperidin-4-yl)acetonitrile, appearance: white to light yellow solid or powder, melting point: 225.6°C to 228.9°C (differential scanning calorimetry-onset (DSC onset) value), and solubility: practically insoluble in water. The present application does not specifically limit the crystalline form of the compound VVN461, as long as it is possible to fulfill the purpose of the present application, e.g., the compound VVN46 is of crystalline form A (X-ray powder diffraction (XRDP) method).

The inventor found that by regulating the content of the active ingredient within the above range and regulating the pH buffering agent within the above low concentration range, the buffering capacity of the ophthalmic composition according to the present application can be made to match the buffering capacity of the tears, and thus obtained ophthalmic composition is tear friendly and comfortable. Moreover, the average residence time of the active ingredient of the present application can be increased, while the bioavailability of the active ingredient is improved.

In the present application, by regulating the content of the tonicity agent or osmolyte within the above range, using an content equivalent to 0.9% sodium chloride solution or 2.7% glycerol solution, the osmolality of the ophthalmic composition according to the present application is brought close to the osmolality of normal tear fluid (270 mOsmol/Kg to 310 mOsmol/Kg), thus greatly reducing the discomfort of the patient when using the ophthalmic composition. In the present application, if the ophthalmic composition is simply formulated using water for injection without the addition of an tonicity agent or osmolyte, it will result in hypotonicity, whereas an excessive amount of tonicity agent or osmolyte may form a hypertonic solution, and either hypotonicity or hypertonicity will cause the lens to lose its desired optical parameters. Also, hypertonic solutions can cause stinging, eye irritation, drying of the ocular surface and the like.

The present application provides an ophthalmic composition comprising a JAK inhibitor for topical eye drop administration, which is compatible with the eye and stable, tear friendly and comfortable, can improve the bioavailability of the active ingredient in the ophthalmic composition, can be used for the treatment of a non-infectious inflammatory disease and have good therapeutic effects. Additionally, ocular surface drop administration is non-destructive and non-invasive to ocular tissues, less invasive and with fewer side effects.

In some embodiments of the present application, the pH buffering agent is any one selected from the group consisting of boric acid-borate, citric acid-citrate, acetic acid-sodium acetate, trometamol-hydrochloric acid, sodium bicarbonate and phosphate;
the borate is at least one selected from the group consisting of sodium borate, potassium borate and any suitable hydrate thereof, wherein, exemplarily, the hydrate is such as sodium borate pentahydrate or sodium borate decahydrate;
the citrate is at least one selected from the group consisting of potassium citrate, sodium citrate, disodium hydrogen citrate, sodium dihydrogen citrate, dipotassium hydrogen citrate, potassium dihydrogen citrate and any suitable hydrate thereof, wherein, exemplarily, the hydrate is such as sodium citrate dihydrate or sodium citrate trihydrate; and
the phosphate is at least one selected from the group consisting of disodium hydrogen phosphate, sodium dihydrogen phosphate, dipotassium hydrogen phosphate, potassium dihydrogen phosphate and any suitable hydrate thereof, wherein, exemplarily, the hydrate is such as disodium hydrogen phosphate heptahydrate or disodium hydrogen phosphate dodecahydrate.

Among them, the citric acid and citrate can be used either as a pH buffering agent or as a chelating agent, and the specific role thereof in the ophthalmic composition of the present application can be determined according to the specific formulation.

In some embodiments of the present application, the pH buffering agent is selected from the group consisting of citric acid-citrates; the citrate may be any known citrate; the citric acid-citrate accounts for 0.001 wt% to 2.5 wt%, preferably 0.01 wt% to 1.5 wt%, more preferably 0.1 wt% to 0.25 wt% of the ophthalmic composition. The inventor found that by selecting and using a citric acid-citrate buffering system and regulating the content of the citric acid-citrate within the above range, the citric acid-citrate concentration and the buffering capacity of the tear fluid are matched so as to minimize the irritating and/or uncomfortable experience from the higher ionic strength buffering system. At the same time, the citric acid-citrate buffering system having a higher ionic strength improves the solubility of the active ingredient in the ophthalmic composition of the present application, which leads to a better stability of the ophthalmic composition of the present application.

In some embodiments of the present application, the tonicity agent or osmolyte is an inorganic tonicity agent or osmolyte and/or an organic tonicity agent or osmolyte;
the inorganic tonicity agent or osmolyte is at least one selected from the group consisting of sodium chloride, potassium chloride, calcium chloride, zinc chloride and magnesium chloride; and
the organic tonicity agent or osmolyte is at least one selected from the group consisting of glucose, glycerol, propylene glycol, glycine, diglycine, alanine, taurine, tetrahydromethylpyrimidine carboxylic acid, erythritol, mannitol, sorbitol and trehalose.

Among them, the glycerol, propylene glycol, mannitol, trehalose can be used either as a tonicity agent or osmolyte or as a comforting agent, and its specific role in the ophthalmic compositions of the present application can be determined according to the specific formulation.

In some embodiments of the present application, the solubilizer is a cyclodextrin, and the cyclodextrin is at least one selected from the group consisting of alpha-cyclodextrin, beta-cyclodextrin, gamma-cyclodextrin, sulfobutyl-beta-cyclodextrin, hydroxypropyl-beta-cyclodextrin and hydroxypropyl-gamma-cyclodextrin.

The inventor found that the active ingredients, such as compound VVN461, are lipophilic molecules with low water solubilities, and that by using the solubilizer of the present application and regulating the content of the solubilizer within above range, it is possible to improve the permeability of the ophthalmic composition and to increase the average retention time of the ophthalmic composition on the ocular surface, to increase the bioavailability of the active ingredient, and to ensure that sufficient and stable active ingredients are dissolved in the tear fluid and pass through the tear film barrier, thereby exerting better therapeutic effects. When the content of the solubilizer is too low or too high, the permeability of the active ingredient of the present application is limited. The inventor found that when the content of the solubilizer of the present application meets the above range, the ophthalmic composition of the present application has better permeability.

In some embodiments of the present application, the ophthalmic excipient further comprises a mucoadhesive agent; the mucoadhesive agent is mucoadhesive polymer, which can be at least one selected from the group consisting of polyvinylpyrrolidone, polyvinyl alcohol, methylcellulose, hydroxypropylmethylcellulose, carboxymethylcelluloses of different molecular weights, sodium hyaluronates of different molecular weights, sodium alginate, polyethylene glycol, thiolated polyacrylic acid, poloxamer, poloxamine, Mrij, Brij, cellulose acetate phthalate, hydroxyethyl cellulose, poly(amidoamine) dendrimer, poly(dimethylsiloxane) and hydroxypropyl guar; and
the mucoadhesive agent accounts for 0.001 wt% to 15 wt%, preferably 0.005 wt% to 10 wt%, more preferably 0.01 wt% to 5 wt% of the ophthalmic composition.

Among them, the poloxamer can be used either as a mucoadhesive agent or as a surfactant; the carboxymethylcellulose, sodium hyaluronate, sodium alginate and hydroxyethyl cellulose can be used either as a mucoadhesive agent or as a comforting agent; the polyethylene glycol can be used as a mucoadhesive agent, as a surfactant, or as a comforting agent; and specific role of each ingredient in the ophthalmic composition of the present application can be determined according to the specific formulation.

The inventor found that by using the mucoadhesive agent of the present application and regulating the content of the mucoadhesive agent in the ophthalmic composition within the above ranges, it is possible to reduce interruption of the human tear film and ocular irritation, increase the pre-corneal residence time of a drug in an ocular delivery system, increase the bioavailability of the active ingredient, and reduce ocular irritation and dryness at the same time.

In some embodiments of the present application, the ophthalmic excipient further comprises a surfactant; the surfactant can be at least one selected from the group consisting of sodium dodecyl sulfate, polyethoxylated sorbitan fatty acid ester, polyoxyethylene alkyl ether, polyoxyethylene stearate, poloxamine, sorbitan fatty acid ester, polyethylene glycol, polyethoxylated fatty alcohol, PEG-40 hydrogenated castor oil, docusate sodium, quaternary ammonium compound, C₆-C₂₀ fatty acid, saccharide-fatty acid ester, fatty acid glyceride, polysorbate, poloxamer and tyloxapol; and
the surfactant accounts for 0.01 wt% to 5 wt% of the ophthalmic composition.

In the present application, the surfactant can be a surfactant beneficial to the eye, which can reduce the surface tension of the ophthalmic composition to less than 50 dyes/cm².

In some embodiments of the present application, the ophthalmic excipient further comprises a comforting agent; the comforting agent is at least one selected from the group consisting of polyol, a cellulose derivative, dextran, polyethylene glycol, polysorbate, povidone, trehalose, hyaluronic acid, sodium hyaluronate and sodium alginate;
the polyol is at least one selected from the group consisting of glycerol, propylene glycol, polyvinyl alcohol and mannitol;
the cellulose derivative is at least one selected from the group consisting of hydroxypropylmethylcellulose-E4M, hydroxypropylmethylcellulose-LV, hydroxyethylcellulose, hydroxymethylcellulose, methylcellulose, hemicellulose and ethylcellulose; and
the comforting agent accounts for 0.001 wt% to 15 wt%, preferably 0.01 wt% to 5 wt% of the ophthalmic composition.

In the present application, by adding the comforting agent and regulating the content of the comforting agent within the above range, it is possible to further improve the comfort of the patient when using the ophthalmic composition of the present application.

In the present application, the ophthalmic composition may or may not comprise a preservative. In some embodiments of the present application, the ophthalmic excipient further comprises a preservative; the preservative is at least one selected from the group consisting of benzalkonium chloride, sorbic acid, disodium edetate, boric acid, sodium borate, sodium bisulfate, sodium thiosulfate, ascorbate, urea peroxide, benzalkonium bromide, sodium chlorite and polyquaternium-1; and the preservative accounts for 0.01 wt% to 0.05 wt% of the ophthalmic composition.

In some embodiments of the present application, the ophthalmic excipient further comprises an antioxidant; the antioxidant is at least one selected from the group consisting of sodium thiosulfate, sodium metabisulfite, N-acetylcysteine, butylhydroxyanisole and butylhydroxytoluene; and the antioxidant accounts for 0.01 wt% to 5 wt%, preferably 0.1 wt% to 0.5 wt% of the ophthalmic composition.

In some embodiments of the present application, the ophthalmic excipient further comprises a chelating agent. Preferably, the chelating agent is ocular surface friendly and biodegradable. Exemplarily, the chelating agent is at least one selected from the group consisting of nitrilotriacetic acid, ethylenediamine disuccinic acid, iminodisuccinic acid, methylglycine diacetic acid, L-glutamic acid N,N-diacetic acid, ethylenediamine N,N'-diglutamic acid, ethylenediamine N,N'-bis(malonic acid), 3-hydroxy-2,2-iminodisuccinic acid, 2-hydroxyethyliminodiacetic acid, pyridine-2,6-dicarboxylic acid, diethylenetriaminepentaacetic acid, hydroxyethyldiaminetriacetic acid, 1,2-diaminocyclohexanetetraacetic acid, hydroxyethylaminodiacetic acid, polyphosphate, citric acid and citrate, tartaric acid and tartrate, ethylenediaminetetraacetic acid and ethylenediaminetetraacetic acid disodium and hexametaphosphoric acid alkali metal salt; and the chelating agent accounts for 0.001 wt% to 1 wt%, preferably 0.1 wt% to 0.25 wt% of the ophthalmic composition.

In some embodiments of the present application, the method of preparing the ophthalmic composition comprises the following steps:
adding 75% to 85% of the total amount of water for injection to a container; stirring continuously and adding the solubilizer, stirring until completely dissolved, heating the mixed solution to 65°C to 75°C, adding a JAK inhibitor, stirring for not less than 2 h, cooling down to 20°C to 30°C, and slowly adding the other ophthalmic excipients in turn, and then stirring for not less than 10 min; adding water for injection to a constant volume, and stirring for not less than 15 min; and after sterilizing the resultant solution, obtaining the ophthalmic composition comprising the JAK inhibitor of the present application.

In some embodiments of the present application, the method of preparing the ophthalmic composition comprises the following steps:
adding to a container an amount of water for injection that is 75% to 85% of the total amount of the ophthalmic composition; stirring continuously and adding a solubilizer and a mucoadhesive agent, stirring until completely dissolved, heating the mixed solution to 65°C to 75°C, adding a JAK inhibitor, stirring for not less than 2 h, cooling down to 20°C to 30°C, and slowly adding the other ophthalmic excipients in turn, and then stirring for not less than 10 min; adding water for injection to a constant volume, and stirring for not less than 15 min; and after sterilizing the resultant solution, obtaining the ophthalmic composition comprising the JAK inhibitor of the present application.

The method of preparation of the present application achieves an ophthalmic composition comprising a JAK inhibitor with sterility, desirable stability, and extremely low total impurity content.

In the present application, there is no particular limitation on the method of sterilization in the method of preparation, as long as the purpose of the present application can be achieved. For example, sterilization can be carried out by terminal heat sterilization, filtration sterilization, electron-beam sterilization, UV systems, etc. Specifically, filtration sterilization can be carried out for example by one-step cold filtration using 0.22 µm sterilizing filter.

In the present application, there is no particular limitation on the canning method of the ophthalmic composition comprising the JAK inhibitor, as long as the purpose of the present application can be achieved. For example, it may be a conventional multi-dose vial and a blow-fill-seal (BFS) disposable vial or multi-dose vial.

In the present application, there is no particular limitation on the degree of polymerization or molecular weight of each polymer, as long as the purpose of the present application can be achieved. For example, the polyvinylpyrrolidone can be PVP K30, the hydroxypropylmethylcellulose can be HPMC E4M or HPMC LV, the polyethylene glycol can be PEG300 or PEG400, the poloxamer can be poloxamer 407 or poloxamer 188, the carboxymethylcellulose has a weight average molecular weight of 400,000 to 600,000, and the sodium hyaluronate has a weight average molecular weight of 800,000 to 1,200,000.

A second aspect of the present application provides use of the ophthalmic composition provided according to the first aspect of the present application in the preparation of a medicament for the treatment of a non-infectious inflammatory disease. The inventor found that the JAK inhibitor described herein as an active ingredient is capable of blocking the JAK-STAT pathway and reducing the expression of pro-inflammatory cytokines by inhibiting the activity of JAKs, thereby enabling the ophthalmic composition described herein for use in the treatment of non-infectious inflammatory diseases. Preferably, the JAK inhibitor comprises VVN461, and the ophthalmic composition comprises VVN461, wherein VVN461 acts as an active ingredient with dual JAK1/TYK2 inhibitory effects, and is capable of targeting JAK/TYK-dependent cytokines and inhibiting the JAK/STAT pathway, thereby allowing the treatment of non-infectious inflammatory diseases.

In some embodiments of the present application, the non-infectious inflammatory disease comprises uveitis. The uveitis is at least one selected from the group consisting of anterior uveitis, choroiditis, iridocyclitis, intermediate uveitis, iritis, panuveitis, pars planitis, posterior uveitis and retinitis.

In the present application, the administration and dosage of the ophthalmic composition is one drop/eye once, and 4 to 6 times daily.

In the present application, the ophthalmic composition comprising JAK inhibitors can be used topically in the eyes, ears and nose.

The embodiments of the present application are described more specifically by way of examples as follows.

### Example 1

### Preparation of the ophthalmic composition comprising VVN461

To a container, 80% of the total amount of water for injection was added, and stirred continuously. Sulfobutyl-beta-cyclodextrin was added and stirred until completely dissolved. The mixed solution was heated to 70°C. VVN461 represented by formula I was added, stirred for not less than 2 h, and cooled down to 25°C. The other ophthalmic excipients were slowly added in turn, and then stirred for not less than 10 min. Water for injection was added to bring the batch to weight, and stirring was continued for 20 min. After filtration sterilization by one-step cold filtration through 0.22 µm sterilizing filter, the ophthalmic composition was obtained.

The product parameters (including ingredient, content, pH and osmolality) of the ophthalmic composition obtained in Example 1 are shown in Table 1.

### Example 2

Example 2 was performed in steps similar to Example 1 except that the product parameters were adjusted as shown in Table 1.

### Examples 3 to 4

Examples 3 to 4 were performed in steps similar to Example 1 except that the product parameters were adjusted as shown in Table 2.

### Examples 5 to 11

### Preparation of the ophthalmic compositions comprising VVN461

To a container, 80% of the total amount of water for injection was added and stirred continuously. Hydroxypropyl-beta-cyclodextrin and PEG400 were added, and stirred until completely dissolved. The mixed solution was heated to 70°C. VVN461 represented by formula I was added, stirred for not less than 2 h, and cooled down to 25°C. The other ophthalmic excipients were slowly added in turn, and then stirred for not less than 10 min. Water for injection was added to bring the batch to weight, and stirring was continued for 20 min. After filtration sterilization by one-step cold filtration through 0.22 µm sterilizing filter, the ophthalmic composition was obtained.

The product parameters (including ingredient, content, pH and osmolality) of the ophthalmic compositions obtained in Examples 5 to 11 are shown in Table 3.

### Examples 12 to 17

Examples 12 to 17 were performed in steps similar to Example 5 except that the product parameters were adjusted as shown in Table 4.

### Examples 18 to 23

Examples 18 to 23 were performed in steps similar to Example 5 except that the product parameters were adjusted as shown in Table 5.

### Examples 24 to 29

Examples 24 to 29 were performed in steps similar to Example 5 except that the product parameters were adjusted as shown in Table 6.

### Examples 30 to 35

Examples 30 to 35 were performed in steps similar to Example 5 except that the product parameters were adjusted as shown in Table 7.

### Examples 36 to 39

### Preparation of the ophthalmic compositions comprising VVN461

To a container, 80% of the total amount of water for injection was added, and stirred continuously. Sulfobutyl-beta-cyclodextrin, hydroxypropyl-gamma-cyclodextrin, poloxamer 407 and PVP K90 were added, and stirred until completely dissolved. The mixed solution was heated to 70°C. VVN461 represented by formula I was added, stirred for not less than 2 h, and cooled down to 25°C. The other ophthalmic excipients were slowly added in turn, and then stirred for not less than 10 min. Water for injection was added to bring the batch to weight, and stirring was continued for 20 min. After filtration sterilization by one-step cold filtration through 0.22 µm sterilizing filter, the ophthalmic composition was obtained.

The product parameters (including ingredient, content, pH and osmolality) of the ophthalmic compositions obtained in Examples 36 to 39 are shown in Table 8.

### Example 40

### Preparation of the ophthalmic composition comprising VVN461

To a container, 80% of the total amount of water for injection was added, and stirred continuously. Hydroxypropyl-beta-cyclodextrin, poloxamer 407 and PEG400 were added, and stirred until completely dissolved. The mixed solution was heated up to 70°C. VVN461 represented by formula I was added, stirred for not less than 2 h, and cooled down to 25°C. The other ophthalmic excipients were slowly added in turn, and then stirred for not less than 10 min. Water for injection was added to bring the batch to weight, and stirring was continued for 20 min. After filtration sterilization by one-step cold filtration through 0.22 µm sterilizing filter, the ophthalmic composition was obtained.

The product parameters (including ingredient, content, pH and osmolality) of the ophthalmic composition obtained in Example 40 are shown in Table 9.

### Example 41

Example 41 was performed in steps similar to Example 1 except that the product parameters were adjusted as shown in Table 10.

### Examples 42 to 64

### Preparation of the ophthalmic compositions comprising VVN461

To a container, 80% of the total amount of water for injection was added, and stirred continuously. Hydroxypropyl-beta-cyclodextrin was added, and dissolved visually. After that, poloxamer 188, polyvinyl alcohol, PVP K30, PEG400 and CMC were added, respectively, and stirred until completely dissolved. The mixed solution was heated up to 70°C. VVN461 represented by formula I was added, stirred for not less than 2 h, and cooled down to 25°C. The other ophthalmic excipients were slowly added in turn, and then stirred for not less than 10 min. Water for injection was added to bring the batch to weight, and stirring was continued for 20 min. After filtration sterilization by one-step cold filtration through 0.22 µm sterilizing filter, the ophthalmic composition was obtained.

Among them, the CMC is an aqueous solution of 1 wt% CMC, and the preparation method is as follows: adding 400 mL of water for injection, stirring, and heating up to 85°C, slowly adding 5 g of CMC (with a weight average molecular weight of about 250,000), stirring until there are no white particles and the mixture is transparent, cooling down to room temperature until it is completely dissolved, adding additional water for injection to 500 mL, and stirring well.

The product parameters (including ingredient, content, pH and osmolality) of the ophthalmic compositions obtained in Examples 42 to 64 are shown in Table 11.

**Table 1**

| Ingredient | Example 1 | Example 2 | Function |
|---|---|---|---|
| | Content (wt%) | Content (wt%) | |
| VVN461 | 0.1 | 0.5 | Active Ingredient |
| Sulfobutyl-beta-cyclodextrin | 1.5 | 7.5 | Solubilizer |
| Boric acid | 0.1 | 0.2 | pH buffering agent |
| Sodium borate | 0.005 | 0.002 | |
| Glycerol | 1.92 | 0.4 | Organic tonicity agent or osmolyte |
| Water for Injection | QS to 100 | QS to 100 | Solvent |
| pH | 6.6±0.2 | 6.6±0.2 | |
| Osmolality (mOsmol/Kg) | 270±30 | 270±30 | |

**Table 2**

| Ingredient | Example 3 | Example 4 | Function |
|---|---|---|---|
| | Content (wt%) | Content (wt%) | |
| VVN461 | 0.5 | 1 | Active Ingredient |
| Hydroxypropyl-beta-cyclodextrin | 5.6 | 11 | Solubilizer |
| Citric Acid | 0.02 | 0.02 | pH buffering agent |
| Sodium Citrate | 0.1 | 0.1 | |
| Sodium Chloride | 0.68 | 0.5 | Inorganic tonicity agent or osmolyte |
| Benzalkonium Chloride | 0.02 | 0.02 | Preservative |
| Water for Injection | QS to 100 | QS to 100 | Solvent |
| pH | 5.6±0.2 | 5.6±0.2 | |
| Osmolality (mOsmol/Kg) | 270±30 | 270±30 | |

**Table 3**

| Ingredient | Example 5 | Example 6 | Example 7 | Example 8 | Example 9 | Example 10 | Example 11 | Function |
|---|---|---|---|---|---|---|---|---|
| | Content (wt%) | Content (wt%) | Content (wt%) | Content (wt%) | Content (wt%) | Content (wt%) | Content (wt%) | |
| VVN461 | 0.1 | 0.1 | 0.1 | 0.1 | 0.1 | 0.1 | 0.1 | Active Ingredient |
| Hydroxyprop yl-beta-cyclodextrin | 1.4 | 1.4 | 1.4 | 1.4 | 1.4 | 1.4 | 1.4 | Solubilizer |
| Citric Acid | 0.2 | 0.02 | 0.02 | 0.02 | 0.02 | 0.02 | 0.02 | pH buffering agent / Chelating Agent |
| Sodium Citrate | 1.0 | 0.1 | 0.1 | 0.1 | 0.1 | 0.1 | 0.1 | |
| Sodium Chloride | 0.50 | 0.77 | 0.77 | 0.77 | 0.77 | 0.77 | 0.77 | Inorganic tonicity agent or osmolyte |
| Benzalkoniu m Chloride | 0.02 | 0.02 | 0.02 | 0.02 | 0.02 | 0.02 | 0.02 | Preservative |
| PEG 400 | 0 | 0.01 | 0.05 | 0.1 | 0.25 | 0.5 | 1.0 | Mucoadhesi ve Agent / Comforting Agent / Surfactant |
| Water for Injection | QS to 100 | QS to 100 | QS to 100 | QS to 100 | QS to 100 | QS to 100 | QS to 100 | Solvent |
| pH | 5.6±0.2 | 5.6±0.2 | 5.6±0.2 | 5.6±0.2 | 5.6±0.2 | 5.6±0.2 | 5.6±0.2 | |
| Osmolality (mOsmol/Kg) | 270±30 | 270±30 | 270±30 | 270±30 | 270±30 | 270±30 | 270±30 | |

**Table 4**

| Ingredient | Example 12 | Example 13 | Example 14 | Example 15 | Example 16 | Example 17 | Function |
|---|---|---|---|---|---|---|---|
| | Content (wt%) | Content (wt%) | Content (wt%) | Content (wt%) | Content (wt%) | Content (wt%) | |
| VVN461 | 0.25 | 0.25 | 0.25 | 0.25 | 0.25 | 0.25 | Active Ingredient |
| Hydroxypropyl-beta-cyclodextrin | 3.5 | 3.5 | 3.5 | 3.5 | 3.5 | 3.5 | Solubilizer |
| Citric Acid | 0.02 | 0.02 | 0.02 | 0.02 | 0.02 | 0.02 | pH buffering agent / Chelating Agent |
| Sodium Citrate | 0.1 | 0.1 | 0.1 | 0.1 | 0.1 | 0.1 | |
| Sodium Chloride | 0.72 | 0.72 | 0.72 | 0.72 | 0.72 | 0.72 | Inorganic tonicity agent or osmolyte |
| Benzalkonium Chloride | 0.02 | 0.02 | 0.02 | 0.02 | 0.02 | 0.02 | Preservative |
| PEG 400 | 0 | 0.01 | 0.05 | 0.1 | 0.5 | 1.0 | Mucoadhesive Agent / Comforting Agent / Surfactant |
| Water for Injection | QS to 100 | QS to 100 | QS to 100 | QS to 100 | QS to 100 | QS to 100 | Solvent |
| pH | 5.6±0.2 | 5.6±0.2 | 5.6±0.2 | 5.6±0.2 | 5.6±0.2 | 5.6±0.2 | |
| Osmolality (mOsmol/Kg) | 270±30 | 270±30 | 270±30 | 270±30 | 270±30 | 270±30 | |

**Table 5**

| Ingredient | Example 18 | Example 19 | Example 20 | Example 21 | Example 22 | Example 23 | Function |
|---|---|---|---|---|---|---|---|
| | Content (wt%) | Content (wt%) | Content (wt%) | Content (wt%) | Content (wt%) | Content (wt%) | |
| VVN461 | 0.5 | 0.5 | 0.5 | 0.5 | 0.5 | 0.5 | Active Ingredient |
| Hydroxypropyl-beta-cyclodextrin | 6 | 6 | 6 | 6 | 6 | 6 | Solubilizer |
| Citric Acid | 0.02 | 0.02 | 0.02 | 0.02 | 0.02 | 0.02 | pH buffering agent / Chelating Agent |
| Sodium Citrate | 0.08 | 0.08 | 0.08 | 0.08 | 0.08 | 0.08 | |
| Sodium Chloride | 0.61 | 0.61 | 0.61 | 0.61 | 0.61 | 0.61 | Inorganic tonicity agent or osmolyte |
| Benzalkonium Chloride | 0.02 | 0.02 | 0.02 | 0.02 | 0.02 | 0.02 | Preservative |
| PEG 400 | 0 | 0.01 | 0.05 | 0.1 | 0.5 | 1.0 | Mucoadhesive Agent / Comforting |
| | | | | | | | Agent / Surfactant |
| Water for Injection | QS to 100 | QS to 100 | QS to 100 | QS to 100 | QS to 100 | QS to 100 | Solvent |
| pH | 5.6±0.2 | 5.6±0.2 | 5.6±0.2 | 5.6±0.2 | 5.6±0.2 | 5.6±0.2 | |
| Osmolality (mOsmol/Kg) | 270±30 | 270±30 | 270±30 | 270±30 | 270±30 | 270±30 | |

**Table 6**

| Ingredient | Example 24 | Example 25 | Example 26 | Example 27 | Example 28 | Example 29 | Function |
|---|---|---|---|---|---|---|---|
| | Content (wt%) | Content (wt%) | Content (wt%) | Content (wt%) | Content (wt%) | Content (wt%) | |
| VVN461 | 0.8 | 0.8 | 0.8 | 0.8 | 0.8 | 0.8 | Active Ingredient |
| Hydroxypropyl-beta-cyclodextrin | 9.2 | 9.2 | 9.2 | 9.2 | 9.2 | 9.2 | Solubilizer |
| Citric Acid | 0.02 | 0.02 | 0.02 | 0.02 | 0.02 | 0.02 | pH buffering agent / Chelating Agent |
| Sodium Citrate | 0.08 | 0.08 | 0.08 | 0.08 | 0.08 | 0.08 | |
| Sodium Chloride | 0.5 | 0.5 | 0.5 | 0.5 | 0.5 | 0.5 | Inorganic tonicity agent or osmolyte |
| Benzalkonium Chloride | 0.02 | 0.02 | 0.02 | 0.02 | 0.02 | 0.02 | Preservative |
| PEG 400 | 0 | 0.01 | 0.05 | 0.1 | 0.5 | 1.0 | Mucoadhesive Agent / Comforting Agent / Surfactant |
| Water for Injection | QS to 100 | QS to 100 | QS to 100 | QS to 100 | QS to 100 | QS to 100 | Solvent |
| pH | 5.6±0.2 | 5.6±0.2 | 5.6±0.2 | 5.6±0.2 | 5.6±0.2 | 5.6±0.2 | |
| Osmolality (mOsmol/Kg) | 270±30 | 270±30 | 270±30 | 270±30 | 270±30 | 270±30 | |

**Table 7**

| Ingredient | Example 30 | Example 31 | Example 32 | Example 33 | Example 34 | Example 35 | Function |
|---|---|---|---|---|---|---|---|
| | Content (wt%) | Content (wt%) | Content (wt%) | Content (wt%) | Content (wt%) | Content (wt%) | |
| VVN461 | 1 | 1 | 1 | 1 | 1 | 1 | Active Ingredient |
| Hydroxypropyl-beta- | 11.5 | 11.5 | 11.5 | 11.5 | 11.5 | 11.5 | Solubilizer |
| cyclodextrin | | | | | | | |
| Citric Acid | 0.02 | 0.02 | 0.02 | 0.02 | 0.02 | 0.02 | pH buffering agent / Chelating Agent |
| Sodium Citrate | 0.08 | 0.08 | 0.08 | 0.08 | 0.08 | 0.08 | |
| Sodium Chloride | 0.436 | 0.436 | 0.436 | 0.436 | 0.436 | 0.436 | Inorganic tonicity agent or osmolyte |
| Benzalkonium Chloride | 0.02 | 0.02 | 0.02 | 0.02 | 0.02 | 0.02 | Preservative |
| PEG 400 | 0 | 0.01 | 0.05 | 0.1 | 0.5 | 1.0 | Mucoadhesive Agent / Comforting Agent / Surfactant |
| Water for Injection | QS to 100 | QS to 100 | QS to 100 | QS to 100 | QS to 100 | QS to 100 | Solvent |
| pH | 5.6±0.2 | 5.6±0.2 | 5.6±0.2 | 5.6±0.2 | 5.6±0.2 | 5.6±0.2 | |
| Osmolality (mOsmol/Kg) | 270±30 | 270±30 | 270±30 | 270±30 | 270±30 | 270±30 | |

**Table 8**

| Ingredient | Example 36 | Example 37 | Example 38 | Example 39 | Function |
|---|---|---|---|---|---|
| | Content (wt%) | Content (wt%) | Content (wt%) | Content (wt%) | |
| VVN461 | 0.5 | 0.5 | 0.5 | 0.6 | Active Ingredient |
| Sulfobutyl-beta-cyclodextrin | 7.5 | 7.5 | 7.5 | 7.5 | Solubilizer |
| Hydroxypropyl-gamma-cyclodextrin | 0 | 0 | 0 | 1.5 | |
| Boric Acid | 0.1 | 0.2 | 0.1 | 0.1 | pH buffering agent |
| Sodium Borate | 0.001 | 0.002 | 0.001 | 0.001 | |
| Glycerol | 0.4 | 0.4 | 0.4 | 0.3 | Organic tonicity agent or osmolyte |
| Propylene Glycol | 0 | 0.1 | 0 | 0 | |
| Poloxamer 407 | 0.2 | 0.2 | 0.2 | 0.2 | Mucoadhesive Agent |
| PVP K90 | 1.0 | 0 | 0 | 1.25 | |
| Tyloxapol | 0.05 | 0.05 | 0.05 | 0.05 | Surfactant |
| Benzalkonium Chloride | 0.02 | 0.02 | 0.02 | 0.02 | Preservative |
| Water for Injection | QS to 100 | QS to 100 | QS to 100 | QS to 100 | Solvent |
| pH | 6.6±0.2 | 6.6±0.2 | 6.6±0.2 | 6.6±0.2 | |
| Osmolality (mOsmol/Kg) | 270±30 | 270±30 | 270±30 | 270±30 | |

**Table 9**

| Ingredient | Example 40 | Function |
|---|---|---|
| | Content (wt%) | |
| VVN461 | 1 | Active Ingredient |
| Hydroxypropyl-beta-cyclodextrin | 8.8 | Solubilizer |
| Citric Acid | 0.05 | pH buffering agent / Chelating Agent |
| Sodium Citrate | 0.6 | |
| Glycerol | 1 | Organic tonicity agent or osmolyte |
| Poloxamer 407 | 0.2 | Mucoadhesive Agent / Surfactant |
| PEG 400 | 2 | |
| Benzalkonium Chloride | 0.02 | Preservative |
| Water for Injection | QS to 100 | Solvent |
| pH | 5.6±0.2 | |
| Osmolality (mOsmol/Kg) | 270±30 | |

**Table 10**

| Ingredient | Example 41 | Function |
|---|---|---|
| | Content (wt%) | |
| VVN461 | 0.8 | Active Ingredient |
| Hydroxypropyl-beta-cyclodextrin | 9.2 | Solubilizer |
| Citric Acid | 0.03 | pH buffering agent / Chelating Agent |
| Sodium Citrate | 0.08 | |
| Sodium Chloride | 0.5 | Inorganic tonicity agent or osmolyte |
| Benzalkonium Chloride | 0.02 | Preservative |
| Sodium Thiosulfate | 0.3 | Antioxidant |
| Water for Injection | QS to 100 | Solvent |
| pH | 5.6±0.2 | |
| Osmolality (mOsmol/Kg) | 270±30 | |

**Table 11**

| Function | Active Ingredi ent | Solubilizer | pH buffering agent | | Inorganic tonicity agent or osmolyte | Preservat ive | Mucoadhe sive Agent / Surfactant | Mucoa dhesive Agent | Mucoa dhesive Agent | Mucoadhesi ve Agent / Comforting Agent / Surfactant | Mucoadh esive Agent | pH | Osmola lity (mOsm ol/Kg) |
|---|---|---|---|---|---|---|---|---|---|---|---|---|---|
| Ingredie nt | VVN46 1 | Hydroxypro pyl-beta-cyclodextrin | Citric Acid | Sodium Citrate | Sodium Chloride | Benzalko nium Chloride | Poloxamer 188 | Polyvin yl alcohol | PVP K30 | PEG 400 | CMC | | |
| Example 42 | 1 | 11 | 0.02 | 0.1 | 0.43 | 0.02 | 0.01 | - | - | - | - | | |
| Example 43 | 1 | 11 | 0.02 | 0.1 | 0.43 | 0.02 | 0.05 | - | - | - | - | | |
| Example 44 | 1 | 11 | 0.02 | 0.1 | 0.43 | 0.02 | 0.1 | - | - | - | - | | |
| Example 45 | 1 | 11 | 0.02 | 0.1 | 0.43 | 0.02 | - | 0.01 | - | - | - | | |
| Example 46 | 1 | 11 | 0.02 | 0.1 | 0.43 | 0.02 | - | 0.05 | - | - | - | | |
| Example 47 | 1 | 11 | 0.02 | 0.1 | 0.43 | 0.02 | - | 0.1 | - | - | - | | |
| Example 48 | 1 | 11 | 0.02 | 0.1 | 0.43 | 0.02 | - | 0.5 | - | - | - | 5.6 ± 0.2 | |
| Example 49 | 1 | 11 | 0.02 | 0.1 | 0.43 | 0.02 | - | 1.4 | - | - | - | | 270±30 |
| Example 50 | 1 | 11 | 0.02 | 0.1 | 0.43 | 0.02 | - | - | 0.01 | - | - | | |
| Example 51 | 1 | 11 | 0.02 | 0.1 | 0.43 | 0.02 | - | - | 0.05 | - | - | | |
| Example 52 | 1 | 11 | 0.02 | 0.1 | 0.43 | 0.02 | - | - | 0.1 | - | - | | |
| Example 53 | 1 | 11 | 0.02 | 0.1 | 0.43 | 0.02 | - | - | 0.5 | - | - | | |
| Example 54 | 1 | 11 | 0.02 | 0.1 | 0.43 | 0.02 | - | - | 2.0 | - | - | | |
| Example 55 | 1 | 11 | 0.02 | 0.1 | 0.40 | 0.02 | - | - | 8 | - | - | | |
| Example 56 | 1 | 11 | 0.02 | 0.1 | 0.43 | 0.02 | - | - | - | 0.01 | - | | |
| Example 57 | 1 | 11 | 0.02 | 0.1 | 0.43 | 0.02 | - | - | - | 0.05 | - | | |
| Example 58 | 1 | 11 | 0.02 | 0.1 | 0.43 | 0.02 | - | - | - | 0.1 | - | | |
| Example 59 | 1 | 11 | 0.02 | 0.1 | 0.40 | 0.02 | - | - | - | 0.5 | - | | |
| Example 60 | 1 | 11 | 0.02 | 0.1 | 0.23 | 0.02 | - | - | - | 2.5 | - | | |
| Example 61 | 1 | 11 | 0.02 | 0.1 | 0.43 | 0.02 | - | - | - | - | 0.01 | | |
| Example 62 | 1 | 11 | 0.02 | 0.1 | 0.43 | 0.02 | - | - | - | - | 0.05 | | |
| Example 63 | 1 | 11 | 0.025 | 0.1 | 0.43 | 0.02 | - | - | - | - | 0.1 | | |
| Example 64 | 1 | 11 | 0.03 | 0.1 | 0.43 | 0.02 | - | - | - | - | 0.5 | | |

| | | | | | | | | | | | | | |
|---|---|---|---|---|---|---|---|---|---|---|---|---|---|
| Note: "-" in Table 11 indicates that the corresponding ingredient is not added. | | | | | | | | | | | | | |

### Stability test:

The prepared ophthalmic compositions comprising VVN461 were canned in multi-dose bottles (5 mL) and stored at 25°C ± 2°C/40% ± 5% RH and 40°C ± 2°C/25% ± 5% RH, respectively, and the stability tests of the ophthalmic compositions were carried out after 1 month and 11 months of storage. The results were as shown in Table 12, where the total impurity assay of the ophthalmic compositions were the sum of the impurity assay obtained by high performance liquid chromatography (HPLC) method.

**Table 12**

| Stability Test | | Acceptance Criteria | Example 29 | | | |
|---|---|---|---|---|---|---|
| Storage Duration | | - | 1 Month | | 11 Months | |
| Storage Condition | | | 25°C±2°C/40% ±5%RH | 40°C±2°C/25% ±5%RH | 25°C±2°C/40% ±5%RH | 40°C±2°C/25% ±5%RH |
| Appearance | | Colorless to light yellow clear solution | Colorless clear solution | Light yellow clear solution | Colorless clear solution | Light yellow clear solution |
| pH Value | | 4.5 to 6.5 | 5.60 | 5.61 | 5.54 | 5.56 |
| Osmolality (mOsmol/Kg) | | 250 to 380 | 270 | 272 | 275 | 298* |
| Re lat ed Ing red ien ts | Individua 1 Impurity (%) | - | RRT 1.49=0.37 | RRT 1.49=0.33 | RRT 0.17=0.05 | RRT 0.17=0.05 |
| | | | | | | RRT 0.96=0.25 |
| | | | | | RRT 0.96=0.06 | RRT 1.23=0.06 |
| | | | | | RRT 1.57=0.19 | RRT 1.57=0.08 |
| | Total Impurity (%) | - | 0.37 | 0.33 | 0.30 | 0.44 |
| Assay (%) | | - | 106.0 | 107.1 | 100.3 | 107.7 |

| | | | | | | |
|---|---|---|---|---|---|---|
| Note: "-" in Table 12 indicates that the corresponding parameter does not exist. *: Since the eye drop bottle is a low density polyethylene bottle, which is a semi-permeable material, there is a water loss when it is placed at 40°C for 11 months, and thus the osmolality will become higher. | | | | | | |

From the stability data in Table 11, it can be seen that the ophthalmic compositions prepared by the preparation method of the present application still have good stability and extremely low impurity content after storage for up to 11 months at 25°C ± 2°C/40% ± 5% RH and 40°C ± 2°C/25% ± 5% RH. In this regard, the relative retention time (RRT) of the individual impurity in Table 12 is the ratio of the retention time of the individual impurity to the retention time of VVN461 represented by Formula I.

### The effect of ophthalmic compositions on experimental autoimmune uveitis

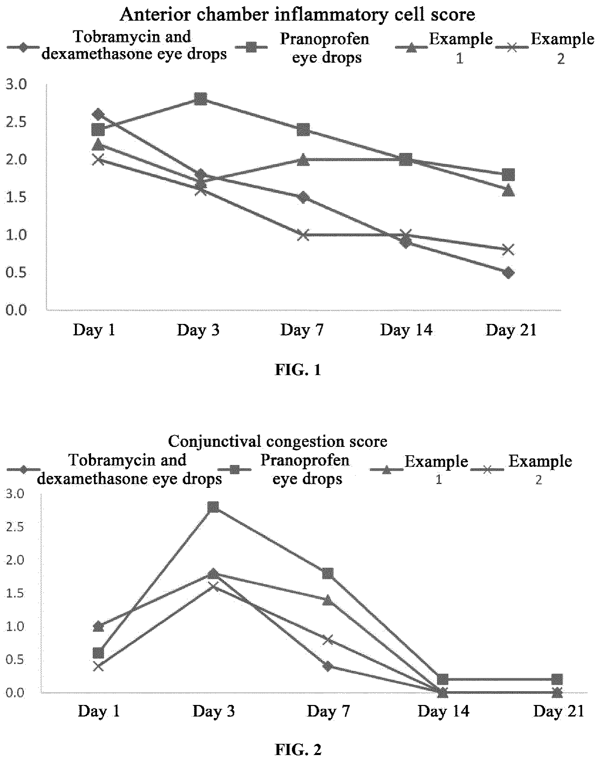

A Dutch rabbit model of anterior uveitis was established to evaluate the efficacy and safety of the ophthalmic compositions. The ophthalmic composition of Example 1, the ophthalmic composition of Example 2, pranoprofen eye drops, and tobramycin and dexamethasone eye drops were administered topically (as eye drops) in one eye of each animal, six times per day, for 21 consecutive days; and the other eye was not treated to serve as a control. The anterior chamber inflammatory cell score and conjunctival congestion score were performed on day 1, day 3, day 7, day 14 and day 21 after administration. The anterior chamber inflammatory cell score criteria are shown in Table 13, and the conjunctival congestion score criteria are shown in Table 14. The results of the anterior chamber inflammatory cell score are shown in FIG. 1, and the results of the conjunctival congestion score are shown in FIG. 2. Based on the results in FIGs. 1 and 2, it can be seen that the ophthalmic compositions of the present application comprising VVN461 for the treatment of experimental autoimmune uveitis are more effective (anterior chamber inflammatory cells were significantly reduced), and the side effects are small (the conjunctival congestion scores were significantly reduced). Their effects were comparable to that of tobramycin and dexamethasone, and better than that of pranoprofen. The above results indicate that the ophthalmic compositions comprising JAK inhibitors using the composition and content of the present application have the potential to replace corticosteroids and can be used for the treatment of non-infectious inflammatory diseases, such as anterior uveitis, and result in better therapeutic effects and fewer side effects.

**Table 13 Anterior chamber inflammatory cell score criteria**

| Score | Severity |
|---|---|
| 0 | No Inflammatory Cell |
| ½ | 1 to 5 Inflammatory Cells |
| 1 | 6 to 15 Inflammatory Cells |
| 2 | 16 to 25 Inflammatory Cells |
| 3 | 26 to 50 Inflammatory Cells |
| 4 | > 50 Inflammatory Cells |

**Table 14 Conjunctival congestion score criteria**

| Score | Indicator |
|---|---|
| 0 | No Congestion |
| 1 | Very Mild Congestion |
| 2 | Mild Congestion |
| 3 | Moderate Congestion |
| 4 | Severe Congestion |

### Mechanism of action of VVN461 for treatment of non-infectious inflammatory disease

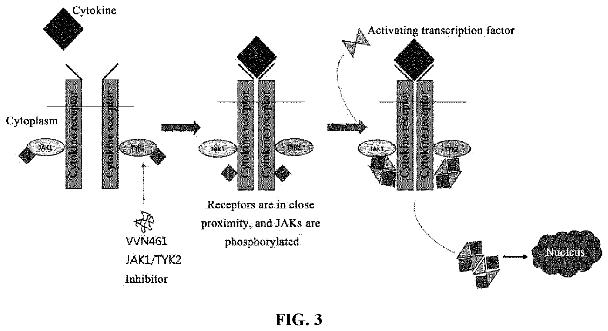

The inventor investigated the mechanism of action of VVN461 and found that VVN461 is a small molecule drug with a dual mode of action as a dual JAK1/TYK2 inhibitor. JAK- or TYK-dependent cytokines are associated with non-infectious inflammatory diseases (e.g., uveitis or other ocular diseases), which is a potential therapeutic target for immune-mediated uveitis. VVN461 can target both Janus kinase 1 (JAK1) and tyrosine kinase 2 (TYK2), target JAK/TYK-dependent cytokines, and inhibit Janus kinase (JAK)/signal transducer and activation of transcription (STAT) pathway, and thus can be used for the treatment of non-infectious inflammatory diseases such as uveitis. As shown in FIG. 3, pro-inflammatory cytokines signal through the JAK/STAT pathway, and when cytokines bind to cytokine receptors in close proximity, JAKs become phosphorylated, and then the tyrosine residues on the intracellular structural domains of the cytokine receptor are phosphorylated. These phosphorylated receptor residues that act as binding sites for STAT proteins are activated by the phosphorylation of the JAKs, so that STAT proteins are dimerized and translocated to the nucleus of the cell, thereby bind to specific DNA sites and regulate gene expression, which in turn leads to inflammation. In contrast, VVN461 of the present application can target JAK/TYK-dependent cytokines and inhibit the JAK/STAT pathway, and thus can be used for the treatment of non-infectious inflammatory diseases such as uveitis.

It should be noted that in this application, the terms "comprise", "include", or any other variation thereof are intended to cover non-exclusive inclusions, so that a process, method or item that includes a series of elements not only includes those elements, but also other elements that are not explicitly listed, or also include elements inherent in such a process, method or item.

The above are only preferable embodiments of the present invention and are not used to limit the scope of protection of the present application. Any modifications, equivalent replacements, improvements, etc., made within the spirit and principles of the present application shall be included in the scope of protection of the present invention.

## Claims

1. An ophthalmic composition comprising an active ingredient and an ophthalmic excipient, wherein the active ingredient is a JAK inhibitor, and the JAK inhibitor is at least one selected from the group consisting of VVN461, tofacitinib, ruxolitinib, baricitinib, peficitinib, delgocitinib, upadacitinib, filgotinib, abrocitinib, deucravacitinib, ritlecitinib, brepocitinib, jaktinib, ivarmacitinib, itacitinib, golidocitinib, KL130008, TLL018 and LNK01001; preferably VVN461; the VVN461 is represented by formula I;
the active ingredient accounts for 0.01 wt% to 5 wt%, preferably 0.1 wt% to 2 wt%, more preferably 0.1 wt% to 1 wt% of the ophthalmic composition; 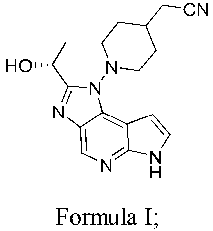
the ophthalmic excipient comprises a pH buffering agent, an tonicity agent or osmolyte, a solubilizer and water for injection; wherein the pH buffering agent accounts for 0.001 wt% to 2.5 wt%, preferably 0.01 wt% to 1.5 wt%, more preferably 0.1 wt% to 0.25 wt% of the ophthalmic composition;
the tonicity agent or osmolyte accounts for 0.01 wt% to 2.5 wt%, preferably 0.2 wt% to 2 wt% of the ophthalmic composition;
the solubilizer accounts for 0.5 wt% to 15 wt%, preferably 1 wt% to 12 wt% of the ophthalmic composition; and
the ophthalmic composition has a pH of 4 to 8, preferably 5 to 7; and an osmolality of 200 mOsmo/Kg to 400 mOsmo/Kg, preferably 240 mOsmo/Kg to 380 mOsmo/Kg, more preferably 240 mOsmo/Kg to 320 mOsmol/Kg.

2. The ophthalmic composition according to claim 1, wherein the pH buffering agent is any one selected from the group consisting of boric acid-borate, citric acid-citrate, acetic acid-sodium acetate, trometamol-hydrochloric acid, sodium bicarbonate and phosphate;
the borate is at least one selected from the group consisting of sodium borate, potassium borate and hydrate thereof;
the citrate is at least one selected from the group consisting of potassium citrate, sodium citrate, disodium hydrogen citrate, sodium dihydrogen citrate, dipotassium hydrogen citrate, potassium dihydrogen citrate and hydrate thereof; and
the phosphate is at least one selected from the group consisting of disodium hydrogen phosphate, sodium dihydrogen phosphate, dipotassium hydrogen phosphate, potassium dihydrogen phosphate and hydrate thereof.

3. The ophthalmic composition according to claim 1, wherein the tonicity agent or osmolyte is an inorganic tonicity agent or osmolyte and/or an organic tonicity agent or osmolyte;
the inorganic tonicity agent or osmolyte is at least one selected from the group consisting of sodium chloride, potassium chloride, calcium chloride, zinc chloride and magnesium chloride; and
the organic tonicity agent or osmolyte is at least one selected from the group consisting of glucose, glycerol, propylene glycol, glycine, diglycine, alanine, taurine, tetrahydromethylpyrimidine carboxylic acid, erythritol, mannitol, sorbitol and trehalose.

4. The ophthalmic composition according to claim 1, wherein the solubilizer is a cyclodextrin, and the cyclodextrin is at least one selected from the group consisting of alpha-cyclodextrin, beta-cyclodextrin, gamma-cyclodextrin, sulfobutyl-beta-cyclodextrin, hydroxypropyl-beta-cyclodextrin and hydroxypropyl-gamma-cyclodextrin.

5. The ophthalmic composition according to any one of claims 1 to 4, wherein the ophthalmic excipient further comprises a mucoadhesive agent; the mucoadhesive agent is at least one selected from the group consisting of polyvinylpyrrolidone, polyvinyl alcohol, methylcellulose, hydroxypropylmethylcellulose, carboxymethylcellulose, sodium hyaluronate, sodium alginate, polyethylene glycol, thiolated polyacrylic acid, poloxamer, poloxamine, Mrij, Brij, cellulose acetate phthalate, hydroxyethyl cellulose, poly(amidoamine) dendrimer, poly(dimethylsiloxane) and hydroxypropyl guar; and
the mucoadhesive agent accounts for 0.001 wt% to 15 wt%, preferably 0.005 wt% to 10 wt%, more preferably 0.01 wt% to 5 wt% of the ophthalmic composition.

6. The ophthalmic composition according to any one of claims 1 to 4, wherein the ophthalmic excipient further comprises a surfactant; the surfactant is at least one selected from the group consisting of sodium dodecyl sulfate, polyethoxylated sorbitan fatty acid ester, polyoxyethylene alkyl ether, polyoxyethylene stearate, poloxamine, sorbitan fatty acid ester, polyethylene glycol, polyethoxylated fatty alcohol, PEG-40 hydrogenated castor oil, docusate sodium, quaternary ammonium compound, C₆-C₂₀ fatty acid, saccharide-fatty acid ester, fatty acid glyceride, polysorbate, poloxamer and tyloxapol; and
the surfactant accounts for 0.01 wt% to 5 wt% of the ophthalmic composition.

7. The ophthalmic composition according to any one of claims 1 to 4, wherein the ophthalmic excipient further comprises a comforting agent; the comforting agent is at least one selected from the group consisting of polyol, cellulose derivative, dextran, polyethylene glycol, polysorbate, povidone, trehalose, hyaluronic acid, sodium hyaluronate and sodium alginate;
the polyol is at least one selected from the group consisting of glycerol, propylene glycol, polyvinyl alcohol and mannitol;
the cellulose derivative is at least one selected from the group consisting of hydroxypropylmethylcellulose-E4M, hydroxypropylmethylcellulose-LV, hydroxyethylcellulose, hydroxymethylcellulose, methylcellulose, hemicellulose and ethylcellulose; and
the comforting agent accounts for 0.001 wt% to 15 wt%, preferably 0.01 wt% to 5 wt% of the ophthalmic composition.

8. The ophthalmic composition according to any one of claims 1 to 4, wherein the ophthalmic excipient further comprises a preservative; the preservative is at least one selected from the group consisting of benzalkonium chloride, sorbic acid, disodium edetate, boric acid, sodium borate, sodium bisulfate, sodium thiosulfate, ascorbate, urea peroxide, benzalkonium bromide, sodium chlorite and polyquaternium-1; and
the preservative accounts for 0.01 wt% to 0.05 wt% of the ophthalmic composition.

9. The ophthalmic composition according to any one of claims 1 to 4, the ophthalmic excipient further comprises an antioxidant; the antioxidant is at least one selected from the group consisting of sodium thiosulfate, sodium metabisulfite, N-acetylcysteine, butylhydroxyanisole and butylhydroxytoluene; and
the antioxidant accounts for 0.01 wt% to 5 wt%, preferably 0.1 wt% to 0.5 wt% of the ophthalmic composition.

10. The ophthalmic composition according to any one of claims 1 to 4, wherein the ophthalmic excipient further comprises a chelating agent; the chelating agent is at least one selected from the group consisting of nitrilotriacetic acid, ethylenediamine disuccinic acid, iminodisuccinic acid, methylglycine diacetic acid, L-glutamic acid N,N-diacetic acid, ethylenediamine N,N'-diglutamic acid, ethylenediamine N,N'-bis(malonic acid), 3-hydroxy-2,2-iminodisuccinic acid, 2-hydroxyethyliminodiacetic acid, pyridine-2,6-dicarboxylic acid, diethylenetriaminepentaacetic acid, hydroxyethyldiaminetriacetic acid, 1,2-diaminocyclohexanetetraacetic acid, hydroxyethylaminodiacetic acid, polyphosphate, citric acid and citrate, tartaric acid and tartrate, ethylenediaminetetraacetic acid and ethylenediaminetetraacetic acid disodium and hexametaphosphoric acid alkali metal salt; and
the chelating agent accounts for 0.001 wt% to 1 wt%, preferably 0.1 wt% to 0.25 wt% of the ophthalmic composition.

11. Use of the ophthalmic composition according to any one of claims 1 to 10 in the preparation of a medicament for the treatment of a non-infectious inflammatory disease.

12. The use according to claim 11, the non-infectious inflammatory disease comprises uveitis; and the uveitis is at least one selected from the group consisting of anterior uveitis, choroiditis, iridocyclitis, intermediate uveitis, iritis, panuveitis, pars planitis, posterior uveitis and retinitis.
